# EUROPEAN PATENT APPLICATION

(11) **EP 4 850 627 A1**
(43) Date of publication of application: **09.12.2026**
(21) Application number: 25180438.1
(22) Date of filing: 03.06.2025
(51) Int. Cl.: A61B 34/20

(54) **SURGICAL REGISTRATION SYSTEM AND METHOD FOR PERFORMING A DATA REGISTRATION PROCESS IN A SURGICAL REGISTRATION SYSTEM**

(71) Applicant: Stryker European Operations Limited, Carrigtwohill, Co. Cork T45 HX08 (IE)
(72) Inventor: GERST, Maximilian, 79098 Freiburg im Breisgau (DE); HORNECKER, Patrick, 79356 Eichstetten (DE)
(74) Representative: Röthinger, Rainer

(57) **Abstract**

The present disclosure provides a surgical registration system (100) which comprising an optical tracking device (150, 160) associated with a patient, an optical detector unit (110) configured to detect the optical tracking device (150, 160) and to output a signal representative of a position of the optical tracking device (150, 160), a data processing unit (130) configured to determine a breathing movement pattern (410, 420, 510, 520) of the optical tracking device (150, 160) based on the output signal, wherein the breathing movement pattern (410, 420, 510, 520) comprises positional data of the optical tracking device (150, 160) assigned to respective points in time of a time period, and wherein the breathing movement pattern (410, 420, 510, 520) defines a plurality of breathing phases (450, 460, 470, 480) of a breathing cycle. The surgical registration system (100) further comprises a medical imaging unit (120) configured to capture a 3D image of the patient at a specific breathing phase (450, 460, 470, 480) of the breathing cycle. The data processing unit (130) is further configured to perform, at the specific breathing phase (450, 460, 470, 480) of the breathing cycle, a registration process for correlating a position of the 3D image with a corresponding patient's position. The present disclosure also provides a method of performing a data registration process in the surgical registration system.

## Description

### Technical Field

The present disclosure relates to a surgical registration system and a method for performing a data registration process in a surgical registration system.

### Background

In order to precisely visualize the position of a tracked surgical instrument in relation to medical image data of the area to be treated during a surgical procedure, the medical image data must be assigned to a corresponding spatial position of the patient. This assignment process is referred to as "registration" of the image data. In particular, a registration process requires the determination of a transformation between an image coordinate system and a patient coordinate system or another spatial coordinate system.

Conventionally, both the patient's body and various instruments are tracked. Such a tracking enables the relative positions between the tracked patient and one or more tracked instruments to be determined. If the image data are "registered" relative to the spatial position of the patient's body, the position of the tracked instrument can be visualized in the medical image data (e.g., for navigation purposes). Medical image data can be recorded using various known methods (e.g. MRI or CT) and registered in real space.

For example, a known method uses a tracking system with a tracker permanently attached to the patient in combination with a tracker attached to a medical imaging device, for example to a C-arm of an X-ray apparatus. With these two trackers detected by a camera of the tracking system, one may "auto-register" the scanned image data into the patient coordinate system. In particular, for recording the medical image data, the radiation source and the opposite detector of the C-arm rotate around a common axis of rotation. The image recording area recorded in this way lies around an isocenter located on the axis of rotation. Since the relative position of the isocenter to the tracker attached to the C-arm is pre-determined at the factory site, the image data of the patient scan can be registered relative to the position of the patient.

A disadvantage of this kind of "auto-registration" is that not all surgical tracking systems are compatible with all medical imaging devices. To avoid this problem, so-called auto-registration devices have been introduced that allow registration independently of tracking the medical imaging device.

When performing registration using an auto-registration device, the camera of the tracking system tracks both an optical tracker that is permanently attached to the patient and an optical tracker of the auto-registration device, which is also typically only temporally associated with the patient. The auto-registration device is only used during and after the medical image acquisition, e.g. the CT scan, for auto-registering the medical image data into the patient coordinate system. After registration, the auto-registration device can be removed, and further tracking of the patient can be performed using the optical tracker that is permanently attached to the patient. For this, it is necessary to "hand over" the registration from the auto-registration device to the patient tracker.

Successful auto-registration using the auto-registration device as well as subsequent transfer of the registration and further tracking with the patient tracker depend crucially on the spatial relationship between the auto-registration device and the patient anatomy (and thus the patient tracker) being constant, or at least not changing in an undetectable manner. Likewise, also when auto-registering medical image data scanned by a medical imaging device to which a tracker is attached, the accuracy of the registering process is determined by a constant spatial relationship between the patient tracker and the patient's anatomy.

### Summary

There is a need to overcome one or more of the above discussed and other problems in the context of a registration process in a surgical registration system. For example, there is a need to enhance the accuracy of the registration process.

According to an aspect of the present disclosure, a surgical registration system comprises an optical tracking device associated with a patient, an optical detector unit configured to detect the optical tracking device and to output a signal representative of a position of the optical tracking device, a data processing unit configured to determine a breathing movement pattern of the optical tracking device based on the output signal, wherein the breathing movement pattern comprises positional data of the optical tracking device assigned to respective points in time of a time period, and wherein the breathing movement pattern defines a plurality of breathing phases of a breathing cycle. The system also comprises a medical imaging unit configured to capture a 3D image of the patient at a specific breathing phase of the breathing cycle, wherein the data processing unit is further configured to perform, at the specific breathing phase of the breathing cycle, a registration process for correlating a position of the 3D image with a corresponding patient's position.

The optical tracking device may be rigidly attached and/or clamped to a patient's bone anatomy. The optical tracking device may be placed on a patient's skin or bone anatomy. The optical tracking device may be pressed into a patient's body, for example via an incision performed beforehand. The optical tracking device may protrude into the patient's body, in particular so as to be close to the patient's bone anatomy. The optical tracking device may be removed before start of the surgery. The optical tracking device may be kept attached to the patient during the surgery so as allow re-registering the 3D image using the optical tracking device.

The optical tracking device may comprise one or more optical markers. The one or more optical markers may be passive optical markers which are configured to reflect light detectable by the optical detection unit, in particular light of a specific wavelength band. The one or more optical markers may be active optical markers which are configured to emit light detectable by the optical detection unit, in particular light of a specific wavelength band. The one or more optical markers may form one or more arrays. The optical detection unit may be a camera.

The breathing movement pattern may define the positional change of the optical tracking device, in particular of the one or more optical markers, caused by a patient's respiration during the surgery. The breathing movement pattern may define a positional deviation from a reference position during a certain time interval. A respiratory or breathing cycle may include an inhaling phase, the phase defining the inhaled state, an exhaling phase, and the phase defining the exhaled state. The phases of different breathing cycles may be similar or identical as regards the amplitude of the positional change/deviation and/or their time duration. The amplitude may be a mean positional change or deviation during a specific phase. A plurality of respiratory or breathing cycles may show a repeatable breathing pattern.

The medical imaging unit may be any medical imaging unit configured to capture a 3D image of the patient or at least a patient's body region. The medical imaging unit may be, for example, a scanning unit, e.g. an MRI (Magnetic Resonance Imaging) device or CT (Computer Tomography) device. The medical imaging unit may also be, for example, a fluoroscopy imaging device. The patient's breathing may be stopped during the medical imaging to ensure a good image quality. The image may be captured in a state where the optical tracking device is attached to or held on the patient's body. The image may be captured in a state where tracking of the optical tracking device by the optical detection unit is stopped or interrupted, thereby allowing determining a position of the optical tracking device during the scan. In particular, the position of the optical tracking device during the scan may correspond to the tracked position when tracking has been stopped. The patient's breathing may be automatically stopped by the surgical registration system or manually by a user.

The imaging step by the medical imaging unit may be a pre-operative imaging step or an intra-operative imaging step. The patient and thus also the optical tracking device may be covered by a sterile drape during the imaging step.

The specific breathing phase at which the 3D scan is performed may be predetermined or may be arbitrary. The specific breathing phase may be the exhaled phase, i.e. the phase during the exhaled state, of a breathing cycle.

The registration process may be an initial or pre-operative registration process or a re-registration process during the surgery. During the registration process, the patient and thus also the optical tracking device may be not or no longer covered by a sterile drape. In the registration process, for correlating a position of the 3D image with a corresponding patient's position, the position of the optical tracking device as determined by the data processing unit based on the output signal of the optical detector unit may be correlated to a position of the 3D image.

The surgical registration system may form part of a surgical navigation system where the surgeon is "navigated" to safely guide a surgical tool, in particular a tracked surgical tool, to a surgical site.

According to an example of the aspect of the present disclosure, the data processing unit may be configured to start or to cause indication of a start of the registration process when the positional data of the first breathing movement pattern of the optical tracking device based on a current detection by the optical detector unit corresponds to the positional data of the optical tracking device when capturing the 3D image.

The data processing unit may automatically start the registration process when it is determined that the current patient's respiratory phase corresponds to the respiratory phase at which the 3D scan has been captured. The patient's respiration may not be interrupted during the registration process. In the alternative, the data processing unit may cause indication to start the registration process when it is determined that the current patient's respiratory phase corresponds to the respiratory phase at which the 3D scan has been captured. For example, a feedback may be provided to a user via a user interface when the respiratory state of the image acquisition during the 3D scan is aligned or within a certain threshold of the respiratory state currently detected and determined. The user may then stop the respiration and manually start the registration process. For example, the user interface may be a display and the feedback may be a displayed text. Additionally or alternatively, the feedback may be also a sound provided via a speaker. Generally, every conceivable feedback and user interface is encompassed by the present disclosure.

In a variant of the aspect of the present disclosure, the data processing unit may be configured to determine the specific breathing phase of the breathing cycle by comparing a breathing movement pattern of the optical tracking device based on a previous detection by the optical detector unit with positional data of the optical tracking device when capturing the 3D image.

The breathing movement pattern based on the previous detection by the optical tracking device may have been detected in the course of a usual intra-operative imaging step and before capturing the intra-operative image. The breathing movement pattern may have been stored in a storage unit. The storage unit may be part of the surgical registration system or be an external storage unit. The storage unit may be or comprise a cache. An intra-operative imaging step may be an imaging step right before the start of the surgical procedure, with the optical tracking device already attached to or held on the patient, and/or an imaging step in the course of the surgical procedure. The breathing phase may be the specific breathing phase if the positional data of the breathing movement pattern matches the positional data of the optical tracking device when capturing the 3D image. The breathing movement pattern based on the previous detection by the optical tracking device may be based on a detection in the same patient's state (draped or undraped) as the patient was during the 3D scan. In the alternative, the breathing phase may be the specific breathing phase if the positional data of the breathing phase has a predefined relation such as a predefined offset with respect to the positional data of the optical tracking device determined based on a detection of the optical tracking device when capturing the 3D image. In this case, the breathing movement pattern based on the previous detection by the optical tracking device may be based on a detection in another patient's state (draped or undraped) as the patient was during the 3D scan.

In an aspect of the variant, the positional data at the specific breathing phase of the breathing movement pattern of the optical tracking device based on the previous detection by the optical detector unit may correspond to the positional data of the optical tracking device when capturing the 3D image. Thus, for determining the specific breathing phase, the breathing phase at which the positional data of the breathing movement pattern of the optical tracking device based on the previous detection by the optical detector unit matches the positional data of the optical tracking device when capturing the 3D image may be identified as the specific breathing phase.

In a further variant of the present disclosure, the breathing movement pattern may comprise a draped breathing movement pattern determined in a draped state of the patient and an undraped breathing movement pattern determined in an undraped state of the patient, and wherein the data processing unit may be configured to determine an offset between the draped breathing movement pattern and the undraped breathing movement pattern, wherein the offset is related to a breathing phase of a breathing cycle.

The draped und undraped breathing movement patterns may be similar or the same as regards the time duration of the different breathing phases, but may be distinguished from each other in the amplitude of the positional change, and in particular in the minimum and maximum of the breathing curve. The draped and undraped breathing patterns may be determined to compensate with distorted data due to the draping when relating positional data obtained in a draped state with positional data obtained in an undraped state.

The draped and undraped breathing patterns may be matched by matching the respective breathing phases of the draped and undraped breathing patterns. Matching respective breathing phases may mean aligning the breathing phases in time, so that a correspondence between positional data of a breathing pattern in an undraped state and positional data of a respective breathing pattern in the draped state may be established. The exhaled state or phase marks the lowest point or inflection point in each of the draped und undraped breathing curves, so that this interval may be used to match the two breathing patterns.

For putting the positional data of a breathing pattern in an undraped state and the positional data of a respective breathing pattern in the draped state into relation to each other, the exhaled positions of the draped and undraped state may be used as reference positions. If breathing has been stopped during the detection and the breathing is mostly stopped in the exhaled state, the system may determine the exhaled state and thus the corresponding exhaled position during a breathing cycle by determining a specific time frame during which no breathing movement has been observed. If no breathing movement has been observed, it may be assumed that breathing has been stopped. It is also possible to stop the detection of the respective breathing pattern in the exhaled state, so that the positional data in the exhaled state is the positional data detected at the point in time where the detection has been stopped. The system may use the respective exhaled position as a reference position for all breathing curves detected beforehand and in future.

The offset may describe the difference in the positional data between the draped breathing pattern and the undraped breathing pattern when the breathing patterns have been matched, i.e. at a specific corresponding time period or point in time of a breathing cycle. For example, the offset may be defined by the difference in the detected position of the tracking device when the patient's breathing is in an exhaled state. The offset may describe a correction which may be taken into account to compensate with the difference in the positional data between the draped breathing pattern and the undraped breathing pattern when the breathing patterns have been matched. The offset may be determined for every or a plurality of specific corresponding time periods or points in time of a breathing cycle.

In a modification of the aspect, the data processing unit may be configured to start or to cause indication of a start of the registration process when the positional data of the undraped breathing movement pattern of the optical tracking device based on a current detection by the optical detector unit corresponds to corrected positional data of the optical tracking device. The corrected positional data may correspond to the positional data of the optical tracking device in the draped state of the patient when capturing the 3D image corrected by the offset between the draped breathing movement pattern and the undraped breathing movement pattern related to the specific breathing phase of the breathing cycle.

In the modification, the positional data during the 3D scan, which is performed in a draped state of the patient, may be corrected by a predetermined offset between the draped and undraped breathing movement patterns at a specific point in time or time period of a breathing cycle. The specific point in time or time period of a breathing cycle may correspond to the breathing state or phase at or during which the 3D scan has been performed. The registration process may be started when the currently determined positional data based on the optical tracking device being detected in an undraped state may correspond to the corrected data.

Also in this modification, the data processing unit may automatically start the registration process when it is determined that the current patient's respiratory phase corresponds to the respiratory phase at which the 3D scan has been captured. In the alternative, the data processing unit may cause indication to start the registration process when it is determined that the current patient's respiratory phase corresponds to the respiratory phase at which the 3D scan has been captured. For example, a feedback may be provided to a user via a user interface when the respiratory state of the image acquisition during the 3D scan is aligned or within a certain threshold of the respiratory state currently detected and determined. The user may then stop the respiration and manually start the registration process. For example, the user interface may be a display and the feedback may be a displayed text. Additionally or alternatively, the feedback may be also a sound provided via a speaker. Generally, every conceivable feedback and user interface is encompassed by the present disclosure.

In some variants, the positional data of the optical tracking device when capturing the 3D image corresponds to the positional data based on a previous detection by the optical detector unit at a point in time where a patient's breathing has been stopped. In particular, for performing a 3D scan, the patient's breathing may be interrupted to ensure the best possible image quality. Stopping of the patient's breathing may be automatically detected by the system, and, following this detection, tracking of the tracking device may be automatically stopped. Thus, the positional data at the point in time (or substantially at the point in time) where the breathing has been stopped is the positional data during the 3D scan.

In a further modification, the optical tracking device may comprise an auto-registration device having a plurality of optical fiducial markers forming a first array and a plurality of radiopaque reference markers forming a second array, and wherein the plurality of radiopaque reference markers may be detectable by the scanning device and the plurality of optical fiducial markers may be detectable by the optical detection unit.

In the further modification, the relative position between the plurality of optical fiducial markers (in particular the geometrical centre of the plurality of optical fiducial markers) and the plurality of radiopaque reference markers (in particular the geometrical centre of the plurality of optical fiducial markers) may be a pre-known dimension. Therefore, when determining the positional data of the plurality of optical fiducial markers based on a detection of the plurality of optical fiducial markers in the patient's coordinate system by means of the optical detection unit, it may be possible to derive the positional data of the plurality of radiopaque reference markers in the patient's coordinate system by using the pre-known dimension. This may allow assigning the position of the optical fiducial markers in the patient's coordinate system a corresponding position in the 3D image, i.e. performing a registration process. The auto-registration device may be placed on a patient's body, e.g. on a patient's skin. The auto-registration device may be loosely held at least partially within the patient's body, e.g. in the patient's skin.

The plurality of radiopaque reference markers may be a plurality of radiopaque beads.

In the further modification, the plurality of radiopaque reference markers may be integrated into one or more blades of the auto-registration device that are configured to be pressed into the patient's body for placing the auto-registration device on the patient. One or more incisions may be made in the patient's skin, in particular close to or at the surgical site, which may be used for pressing the blades into the patient's body. The blades with the plurality of radiopaque reference markers may be located close to the anatomy where the surgery is intended to take place, in particular close to the patient's spine. The plurality of optical markers may be located at an end of the auto-registration device opposite to the blades. A part of the auto-registration device may be placed on the patient's skin when the blades are pressed into the patient's body. In particular, a holder of the auto-registration device may be placed on the patient's skin when the blades are pressed into the patient's body.

In addition or in the alternative, the optical tracking device may comprise a patient tracker device having a plurality of optical markers forming a third array and detectable by the optical detection unit. The patient tracker device may be rigidly connected to a patient's bone anatomy, in particular to an exposed bone anatomy for being accessible during the surgery. The plurality of optical markers may be passive optical markers and/or active optical markers.

In the alternative that the optical tracking device comprises both the patient tracker device and the auto-registration device, the breathing movement pattern may comprise a first breathing movement pattern of the auto-registration device and a second breathing movement pattern of the patient tracker device. Furthermore, the first breathing movement pattern may comprise a first draped breathing movement pattern and a first undraped breathing movement pattern, as well as a second draped breathing movement pattern and a second undraped breathing movement pattern.

The above explanations regarding the breathing movement pattern may also apply to each of the first breathing movement pattern and second breathing movement pattern. For example, an offset may be determined between a first draped breathing movement pattern and a first undraped breathing movement pattern, and between a second draped breathing movement pattern and a second undraped breathing movement pattern.

Also in the alternative that the optical tracking device comprises both the patient tracker device and the auto-registration device, a registration process using the auto-registration device and the patient tracker device may be performed. The registration process may comprise an initial registration process using the auto-registration device and one or more re-registration processes using the patient tracker device. Between the initial registration process and the one or more re-registration processes one or more intra-operative 3D images may be captured by the medical imaging unit. Furthermore, before performing or after having performed the one or more re-registration processes, the auto-registration device may be removed from the patient.

In the implementations where the optical tracking device comprises at least the auto-registration device, the registration process may correlate a position of the plurality of radiopaque reference markers forming the second array in the 3D image captured by the medical imaging unit with a position of the plurality of optical fiducial markers forming the first array in the patient's coordinate system determined based on the output signal of the optical detector unit. The respective positions may be correlated by determining the position of the plurality of optical fiducial markers based on a detection by means of the optical detection unit and using a pre-known relative position between the plurality of optical fiducial markers and the radiopaque reference markers. The registration process may be an initial registration process.

In the implementations where the optical tracking device comprises both the auto-registration device and the patient tracker device, the registration process may be or may comprise a registration process which correlates the position of the plurality of radiopaque reference markers forming the second array in the 3D image captured by the medical imaging unit with a position of the plurality of optical markers of the patient tracker device forming the third array determined based on the output signal of the optical detector unit. The registration process may be a re-registration process, in particular an intra-operative registration process. The registration process may be performed after an initial registration process. The respective positions may be correlated by determining the position of the plurality of optical markers in the patient's coordinate system based on a detection by means of the optical detection unit and a determination of a relative position between the plurality of optical markers and the radiopaque reference markers and/or the optical fiducial markers in the patient's coordinate system. Since the position of the optical fiducial markers in the 3D image is known due to the initial registration process, a position of the optical markers of the patient tracker device in the 3D scan may be determined by using the determined relative position between the plurality of optical markers and the radiopaque reference markers and/or the optical fiducial markers in the patient's coordinate system. In particular, for example, the relative position between the plurality of optical markers and the radiopaque reference markers may be determined by using a relative position between the plurality of optical markers and the plurality of optical fiducial markers and a pre-known relative position between the plurality of optical fiducial markers and the plurality of radiopaque reference markers. The relative position between the plurality of optical markers and the plurality of optical fiducial markers may be pre-known or pre-determined or determined based on a detection of the optical markers and the optical fiducial markers by means of the optical detection unit.

In particular, the data processing unit may be configured to determine a relative position between the plurality of optical markers of the patient tracker device and the plurality of optical fiducial markers of the auto-registration device at the specific breathing phase of the breathing cycle at which the 3D scan has been captured. In that way, also when transferring "tracker data" and/or "registration data", a potential positional mismatch in the captured 3D scan of the patient and the positional relationships between the patient's bone anatomy and the/or the auto-registration device and/or the patient tracker device when determining the relative position may be compensated with.

In some implementations, the system may comprise a storing unit configured to store the breathing movement pattern determined by the data processing unit. The storing unit may be or comprise a cache. The stored breathing movement pattern may be a breathing movement pattern for determining the respiratory phase (breathing phase) of a breathing cycle during which the 3D scan has been captured. The breathing movement pattern may comprise a draped and undraped breathing movement pattern. The stored draped and undraped breathing movement patterns may be used to determine the offset.

The present disclosure also provides for a method for performing a registration process in a surgical registration system.

The explanations made above with respect to the surgical registration system also apply to same or corresponding features and aspects of the disclosed method.

In particular, a method of performing a registering process in a surgical registration system is provided which comprises the steps of detecting, by an optical detector unit, an optical tracking device associated with a patient and outputting a signal representative of the position of the optical tracking device, determining, by a data processing unit, a breathing movement pattern of the optical tracking device based on the output signal, wherein the breathing movement pattern comprises positional data of the optical tracking device assigned to respective points in time of a time period, and wherein the breathing movement pattern defines a plurality of breathing phases of a breathing cycle. The method further comprises the steps of capturing, by a medical imaging unit, a 3D image of the patient at a specific breathing phase of the breathing cycle while detecting the optical tracking device, and performing, by the data processing unit, at the specific breathing phase of the breathing cycle, a registration process for correlating a position of the 3D image with a corresponding patient's position.

In a variant of the further aspect, the method may comprise the step of starting or causing indication of a start of the registration process, by the data processing unit, when the positional data of the first breathing movement pattern of the optical tracking device based on a current detection by the optical detector unit corresponds to the positional data of the optical tracking device when capturing the 3D image.

In a further variant of the further aspect, the method may comprise the step of determining, by the data processing unit, the specific breathing phase of the breathing cycle by comparing a breathing movement pattern of the first optical tracking device based on a previous detection by the optical detector unit with the positional data of the optical tracking device when capturing the 3D image.

In a still further variant, the breathing movement pattern may comprise a draped breathing movement pattern determined in a draped state of the patient and an undraped breathing movement pattern determined in an undraped state of the patient, wherein the data processing unit may be configured to determine an offset between the draped breathing movement pattern and the undraped breathing movement pattern, wherein the offset may be related to a breathing phase of a breathing cycle.

In an implementation, the method may comprise the step of starting or causing indication of a start of the registration process, by the data processing unit, when the positional data of the undraped breathing movement pattern of the optical tracking device based on a current detection by the optical detector unit corresponds to corrected positional data of the optical tracking device, wherein the corrected positional data may correspond to the positional data of the optical tracking device when capturing the 3D image corrected by the offset between the draped breathing movement pattern and the undraped breathing movement pattern related to the specific breathing phase of the breathing cycle.

In some implementations, the positional data of the optical tracking device when capturing the 3D image corresponds to the positional data previously detected by the optical detector unit at a point in time where a patient's breathing has been stopped.

In implementations of the method, during the registration process, a position of a plurality of radiopaque reference markers forming a second array in the 3D image captured by the medical imaging unit may be correlated with a position of a plurality of optical fiducial markers forming a first array determined based on the output signal of the optical detector unit, wherein the plurality of radiopaque reference markers may be detectable by the scanning device and the plurality of optical fiducial markers may be detectable by the optical detector unit.

In variants of the implementations, the optical tracking device may comprise an auto-registration device having the plurality of optical fiducial markers forming the first array and the plurality of radiopaque reference markers forming the second array.

In further variants of the implementations, during the registration process, the position of the plurality of radiopaque reference markers forming the second array in the 3D image captured by the medical imaging unit may be correlated with a position of a plurality of optical markers forming a third array determined based on the output signal of the optical detector unit, wherein the plurality of optical markers forming the third array may be detectable by the optical detector unit. In some still further variants, the optical tracking device may comprise a patient tracker device having the plurality of optical markers forming the third array.

In variants of the further aspect, the breathing movement pattern may comprise at least one of a first breathing movement pattern of the auto-registration device and a second breathing movement pattern of the patient tracker device.

Thus, when the optical tracking device comprises the auto-registration device, during the registration process, a position of the plurality of radiopaque reference markers forming the second array in the 3D image captured by the medical imaging unit may be correlated with a position of the plurality of optical fiducial markers forming the first array determined based on the output signal of the optical detector unit.

When the optical tracking device comprises both the auto-registration device and the patient tracker device, during the registration process, the position of the plurality of radiopaque reference markers forming the second array in the 3D image captured by the medical imaging unit may be correlated with a position of the plurality of markers of the patient tracker device forming the third array determined based on the output signal of the optical detector unit.

In further variants, the method may comprise the step of storing the breathing movement pattern determined by the data processing unit.

### Brief Description of the Drawings

Further details, advantages and aspects of the present disclosure will become apparent from the following embodiments taken in conjunction with the drawings, wherein:
- Figure 1: schematically shows an example of a surgical registration system according to the present disclosure comprising an exemplary auto-registration device and a patient tracker device;
- Figure 2: schematically shows, in a perspective view, the example of the auto-registration device of Fig. 1 detached from a patient's body;
- Figure 3: shows a workflow comprising a sequence of some steps of a method of performing a data registering process in a surgical registration system;
- Figure 4: shows an example of real breathing data obtained in a laboratory, for the patient tracker device and the auto-registration device;
- Figure 5: shows an abstract representation of breathing movement patterns in an undraped state of the patient, for the patient tracker device and the auto-registration device;
- Figure 6: shows an abstract representation of breathing movement patterns in a draped state of the patient, for the patient tracker device and the auto-registration device;
- Figure 7A: shows the offset between the breathing movement pattern in the draped state and the undraped state of the patient tracker device, after the respective breathing phases of the breathing movement patterns have been matched;
- Figure 7B: shows the offset between the breathing movement pattern in the draped state and the undraped state of the auto-registration device, after the respective breathing phases of the breathing movement patterns have been matched;
- Figure 8: shows the breathing movement patterns of the patient tracker device and the auto-registration device while a 3D image is captured;
- Figure 9: shows the breathing movement patterns of the patient tracker device and the auto-registration device of Figure 8 matched to pre-recorded breathing movement patterns of the patient tracker device and the auto-registration device;
- Figure 10A: schematically illustrates the aspect how the offset at the breathing phase of the 3D scan is used to correlate and compare breathing movement data of the patient tracker device recorded in the draped state and the undraped state; and
- Figure 10B: schematically illustrates the aspect how the offset at the breathing phase of the 3D scan is used to correlate and compare breathing movement data of the auto-registration device recorded in the draped state and the undraped state.

### Detailed Description of the Drawings

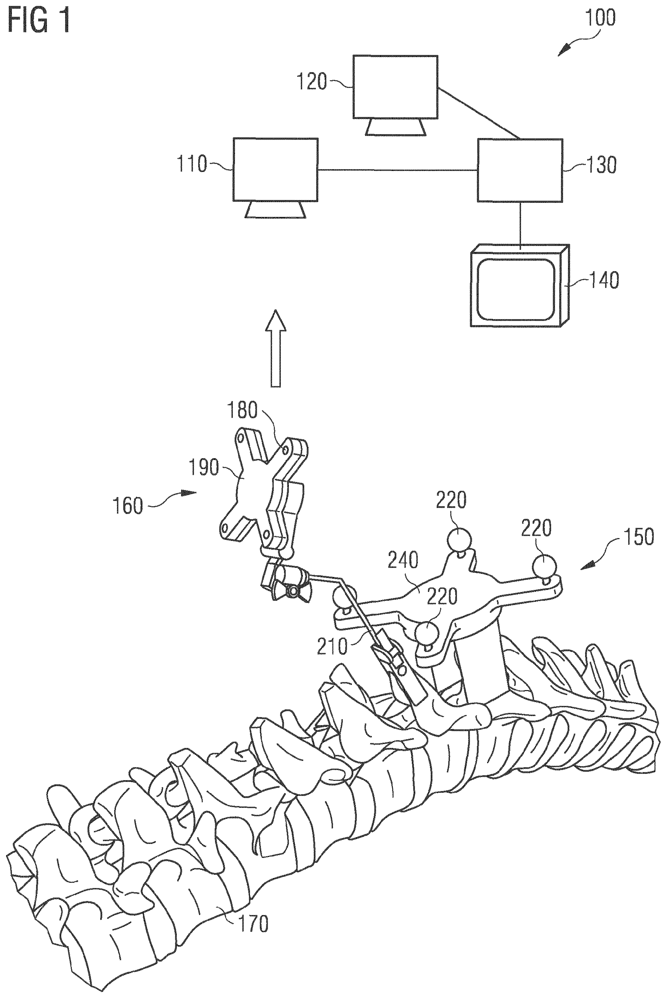

Figure 1 shows an example of surgical registration system 100 according to the present disclosure. The surgical registration system may be part of a surgical navigation system. It is noted that Figure 1 is only a schematic drawing, which does not reproduce the correct relative dimensions between the individual components of the surgical registration system 100. The surgical registration system 100 comprises an optical detector unit 110, a medical imaging unit 120, which is in the present example a scanning unit 120, a data processing unit 130 and an output device 140. The surgical registration system 100 further comprises at least two optical tracking devices, namely an auto-registration device 150 and a patient tracker device 160. The data processing unit 130 may be part of a computer. The surgical registration system 100 may further comprise a storage unit (not shown).

The optical detector unit 110 may be a camera of a surgical tracking system, in particular a stereo camera. The surgical tracking system may be configured to track the position and/or orientation of trackers with optical markers in a tracking coordinate system (e.g., having its origin in a center of an optical sensor of the optical detector unit 110). Such trackers may be integrated in or attached to or held (e.g., placed) on a patient, a surgical tool, the scanning unit 120 (not shown), and so on. The surgical tracking system may be integrated (e.g., as a software routine) into the surgical registration system 100.

Figure 1 shows the auto-registration device 150 held on a patient's body in close proximity to the patient tracker device 160 attached to the patient's spine 170. The patient tracker device 160 comprises a plurality of optical markers 180 (in the present example: four optical markers) and a tracker housing 190. The plurality of optical markers 180 is detectable by the optical detector unit 110 of the surgical registration system 100, e.g. a camera or stereo camera, and forms a third array, i.e. a marker array. In the present embodiment, the optical markers 180 are active optical markers and are thus able to emit light, in particular light with a predetermined wavelength range, for example light in the IR spectrum. In such a case, the surgical registration system 100 is less affected by ambient light. The optical detector unit 110 is configured to generate sensor signals indicative of the detected light. Different sensor signals may be generated for different tracker elements.

The tracker housing 190 has recesses corresponding to the size and shape of the optical markers 180, so that the optical markers 180 lie exposed in the tracker housing 190. The tracker housing 190 with the plurality of optical markers 180 is kept spaced from the patient's spine 170 with a defined distance and orientation by means of a rod 210 attached at one end to the spine 170 and at the opposite end to the tracker housing 190.

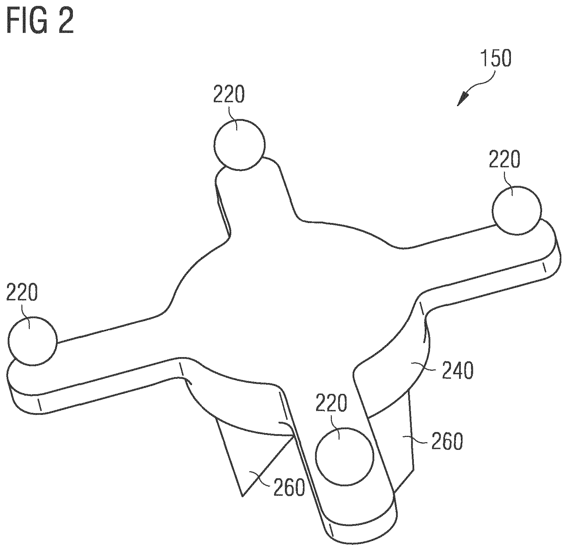

As is seen in Figure 1, the auto-registration device 150 comprises a plurality of optical fiducial markers 220 forming a first array, which are also detectable by the optical detector unit 110 of the surgical registration system 100. In the present embodiment, the auto-registration device 150 comprises four optical fiducial markers 220 forming the first array (i.e. a fiducial array). Furthermore, the optical fiducial markers 220 are passive optical markers which are configured to reflect light, in particular to reflect light with a predetermined wavelength range. In the present embodiment, with further reference to Figure 2, the plurality of optical fiducial markers 220 is attached to a holder 240. In a state where the auto-registration device 150 is hold on or at the patient's body, the fiducial array faces away from the patient's body, in a direction towards the optical detector unit 110.

With further reference to Figure 2, the auto-registration device 150 comprises, in addition to the fiducial array, a plurality of radiopaque reference markers (not shown here). In the present auto-registration device 150, the plurality of radiopaque reference markers is configured as a plurality of radiopaque beads. However, according to the present disclosure, the radiopaque reference markers may have any shape and/or configuration which allows a detection by the scanning unit 120. In particular, the radiopaque beads are detectable by the scanning unit 120 for capturing three-dimensional (3D) medical images and thus can be seen in the scanned medical image data. The radiopaque beads are integrated into one or more blades 260 (in the present example two blades 260) that extend from the holder 240 in a direction opposite to the optical fiducial markers 220. The beads in each of the one or more blades 260 form one or more second arrays, i.e. one or more bead arrays. The one or more blades 260 containing the beads are configured to be pressed into the patient's body (via incision, for example) in close proximity to be worked on during the surgery.

In Figure 1, the skin covering the portions of the spine 170 that are not exposed during the surgery is not illustrated. The patient tracker device 160 may be attached to the spine 170 in an exposed region. The auto-registration device 150 may be placed on the patient's body by making an incision into the patient's skin and pressing the blades 260 into the incision so as to extend close to or into the spine 170. In particular, the holder 250 may be placed on the patient's skin.

The data processing unit 130 is configured to generate, from the signals output by the optical detector unit 110, positional data and to locate the auto-registration device 150 comprising the optical fiducial markers 220 based on the positional data. The data processing unit 130 may be part of the optical detector unit 110 or of a detector system comprising the optical detector unit 110. The data processing unit 130 may integrate algorithms of the tracking system.

The output device 140 may comprise at least one of a display, a speaker, a beamer, and a haptic feedback device. In Figure 1, the output device 140 comprises a display. The output device 140 is configured to output information and/or instructions that result from tracking a localizable surgical tool not shown here (e.g., navigation information and/or navigation instructions), which may be part of the surgical navigation system.

The output information and/or instructions may comprise at least one of a visual display of the tracked surgical tool, a visual display of the tracked surgical tool relative to patient data acquired by the scanning unit 120, and an acoustic signal indicative of a position of the tracked surgical tool (e.g., relative to a virtual boundary with respect to a patient). The instructions may comprise at least one of a visual representation of a target shape, target position and target orientation of the tracked surgical tool, and a voice output guiding the surgeon.

In an aspect of the present disclosure, medical imaging data of a patient's bone anatomy with the auto-registration device 150 held on the patient's body is generated by means of the medical image unit 120, for example a scanning unit configured as a CT system. Since the beads of the blades 260 are radiopaque, the medical imaging data will be representative of the patient's bone anatomy and the radiopaque beads 250.

During a surgical procedure, the medical imaging data thus obtained may be used to display one or more images of the patient's bone anatomy on the visual display of the output device 140. The displayed patient's bone anatomy may be overlaid by a visual display of a tracked surgical tool (or at least a visual display representative of the position thereof) in order to assist the surgeon to navigate the surgical tool relative to the surgical imaging data in the surgical navigation system.

A prerequisite for a precise and reliable navigation during the surgical procedure is that the medical imaging data and the data of the optical detector unit 110 generated during the surgical procedure, based on which the visual display of the tracked surgical tool or a representation of its position is generated, are correlated with each other. That is to say, the surgical navigation system has to "know" which point in the medical image corresponds to which point in a tracking coordinate system in which the optical detector unit 110 tracks the surgical tool. The correlation process is called a registration.

In order to register the scanned medical image data with regard to their spatial position in the coordinate system of the surgical registration system 100, the beads are identified in the scanned image data using image processing. Furthermore, the optical fiducial markers 220 forming the first array are detected by the optical detector unit 110 and a signal representative of the position of the fiducial array is output. The data processing unit 130 determines the position of the fiducial array based on the output signal.

In particular, the data processing unit 130 is configured to generate, from the signals output by the optical detector unit 110, positional data and to locate the auto-registration device 150 comprising the optical fiducial markers 220 based on the positional data. The data processing unit 130 may be part of the optical detector unit 110 or of a detector system comprising the optical detector unit 110. The data processing unit 130 may integrate algorithms of the tracking system.

The radiopaque beads are used to determine the placement of the optical fiducial markers 220 within the scanned volume obtained by the scanned image data. The image data (e.g. as obtained in a DICOM format) may be represented in an image coordinate system (e.g. as defined in the DICOM specifications). In particular, since the radiopaque beads have a known spatial relationship to the fiducial array of the auto-registration device 150 (obtained from the device manufacturer and/or from CAD data) and the position of this fiducial array in space, i.e. in the coordinate system of the optical detector unit 110, is determined, the relation of the optical fiducial markers 220 to the scanned volume can be calculated. In that way, a position in the coordinate system of the optical detector unit 110 can be assigned a respective position in the scanned volume (e.g., the image coordinate system), and vice versa. Such an assignment can take the form of a coordinate system transformation.

After this auto-registration process, the auto-registration device 150 may be removed and further tracking of the patient and/or a re-registration process is performed using the patient tracker device 160 that is permanently attached to the patient during the surgery. For this, a tracker transfer from the auto-registration device 150 to the patient tracker device 160 is necessary which results in having set the optical markers 180 of the patient tracker device 160 in relation to the scanned volume (e.g. the image coordinate system). The optical markers 180 of the patient tracker device 160 can be set in relation to the scanned volume due to the knowledge of the spatial relationship of the fiducial markers 220 to the scanned image data and of the fiducial markers 220 to the optical markers 180. After the tracker transfer, the relative position of the optical markers 180 to the scanned volume is known (e.g. in the form of a coordinate system transformation), which is called a patient's registration process.

Successful auto-registration using the auto-registration device 150 as well as subsequent transfer of the registration and further tracking with the patient tracker device 160 is heavily reliant on the spatial relationship between the fiducial array and the radiopaque bead arrays as well as the relationship between the auto-registration device 150 and the patient's anatomy (and thus the patient tracker device 150). If these spatial relationships are altered, in particular in a way that is undetectable, the surgeon may use an inaccurate map of the patient's anatomy (i.e. the medical image data are not correctly assigned to a respective spatial position), which could lead to serious injury of the patient. The patient's breathing during the surgery is one factor that may alter these spatial relationships.

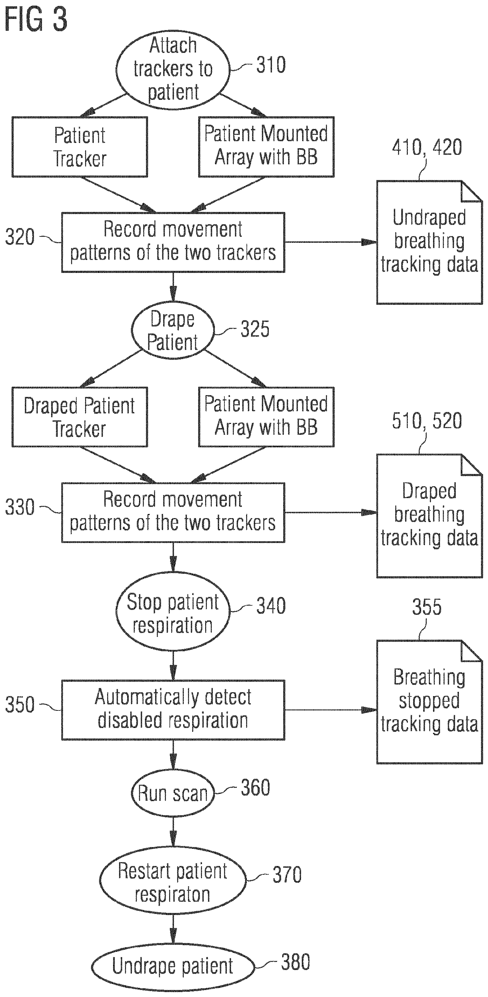

With reference to Figure 3 of the drawings, in a first step 310, the auto-registration device 150 is placed on the patient's body and the patient tracker device 160 is attached to the patient's body, for example to or near to the patient's spine 170, as is exemplarily illustrated in Figure 1. The first step may be performed by a surgeon or another medical person capable and authorized to perform this step. This first step may not form part of the present method for which protection is sought.

In a second step 320, a breathing movement pattern of the fiducial array of the auto-registration device 150 caused by the patient's breathing is recorded during a specific time period and a breathing movement pattern of the marker array of the patient tracker device 160 caused by the patient's breathing is recorded during a specific time period. The specific time periods may be the same or different. The specific time period may be determined by a person operating the optical detector device during the detection process, or may be a predefined specific time period. Both the auto-registration device 150 and the patient tracker device 160 are in an undraped state, i.e. are not covered by a sterile drape. The second step 320 is executed by the surgical registration system 100. The recorded breathing patterns may be stored on a storage unit.

For recording the respective movement pattern, the optical detector unit 110 of the surgical registration system 100, which may be a camera, for example, detects the optical fiducial markers 220 of the fiducial array and outputs signals which are representative of the position of the optical fiducial markers 220 and thus of the fiducial array. The data processing unit 130 may determine a first breathing movement pattern of the optical fiducial markers 220 and thus of the fiducial array (and thus also of the auto-registration device 150) based on the output signals of the optical detector unit 110.

Likewise, the optical detector unit 110 detects the optical markers 180 of the marker array and outputs signals which are representative of the position of the optical markers 180 and thus of the marker array. The data processing unit 130 may determine a second breathing movement pattern of the optical markers 180 and thus of the marker array (and thus also of the patient tracker device 160) based on the output signals of the optical detector unit 110.

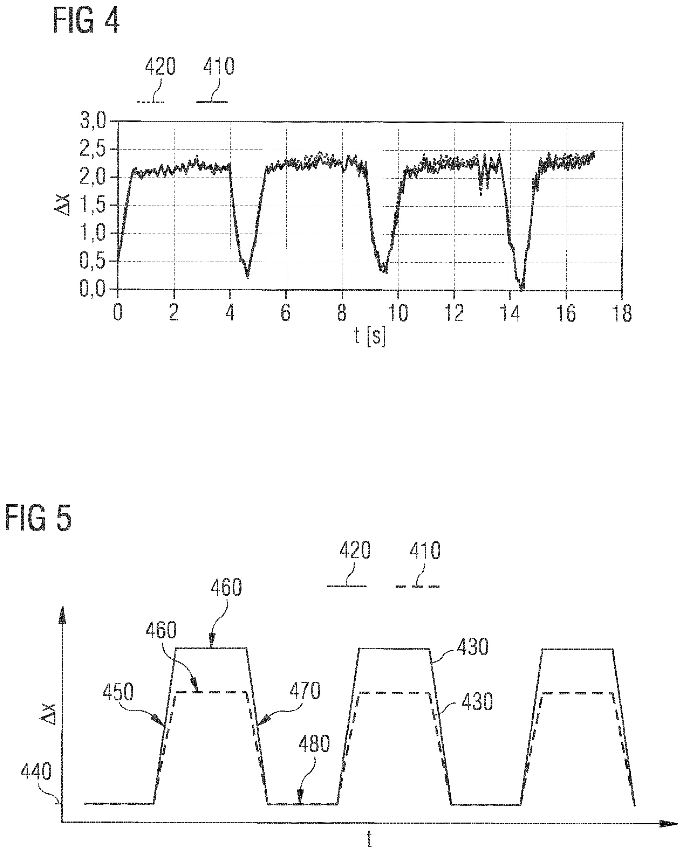

Examples of breathing movement patterns in the undraped state are shown in Fig-ures 4 and 5 of the drawings. Figure 4 shows first and second breathing movement patterns 410, 420 of real breathing data for the fiducial array and the marker array, respectively, obtained in a laboratory. Figure 5 shows an abstract representation of the first and second breathing movement patterns 410, 420 for the fiducial array and the marker array, respectively.

A breathing movement pattern 410, 420 is represented by a breathing curve 430 which shows a positional deviation Δx (i.e. Euclidian distance) from a reference position at Δx=0 in dependence upon the time t. The reference position at Δx=0 may be a predefined reference position. The reference position may be any predetermined position defining a corresponding predetermined breathing state and/or point in time on the breathing curves.

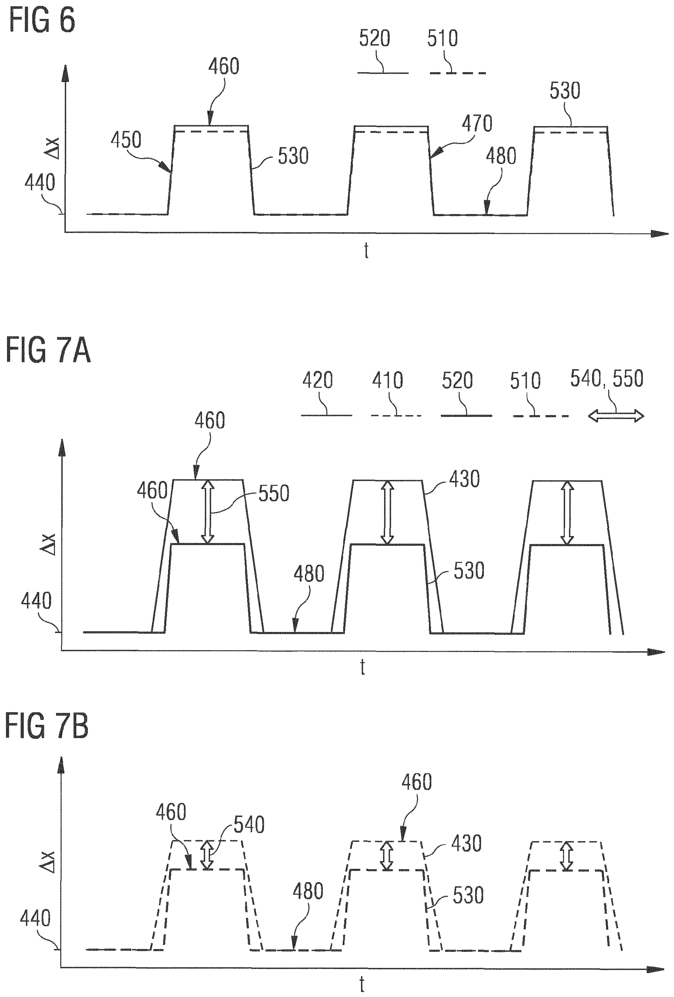

In the present examples, the reference position is a respective position in an exhaled breathing state, in particular a respective minimum position in an exhaled breathing state. A breathing movement pattern 410, 420 defines a plurality of breathing phases of a breathing cycle, wherein the breathing movement pattern may encompass a plurality of breathing cycles, as is seen in Figures 4 and 6. A breathing cycle comprises an inhaling phase 450, a state or phase 460 where the lungs are full or are substantially full of air (inhaled phase or state), and exhaling phase 470, and a state or phase 480 where there is no or substantially no air in the lungs (exhaled state or phase). The states/phases 450, 460, 470, 480 are characterised by respective positions and/or (mean) deviations Δx from the reference position at Δx=0 and/or how much the respective position has changed over a certain time period.

To determine the exhaled phase 480 during a breathing cycle, the surgical registration system 100, in particular the data processing unit 130 of the surgical registration system 100, determines that no breathing movement is observed during a specific time period. If no breathing movement is observed, it can be assumed that the patient's breathing has been stopped. Since the breathing is mostly stopped in the exhaled state 480, the data processing unit 130 can derive the position on the breathing curve 430 at the time where the breathing has been stopped, which is the position of the respective array in the exhaled state/phase 480 of the patient's breathing. The position may be different in the draped state and the undraped state, for each of the patient tracker device 160 and the auto-registration device 150. The so determined exhaled position may be set as a reference position 440 for all previous and future breathing curves 430.

As is seen in Figures 4 and 5, the breathing curves 430 of the marker array and the fiducial array show the same characteristics, such as the duration of the plurality of breathing cycles and the duration of the breathing phases 450, 460, 470, 480 of a respective breathing cycle, provided that the anatomical positioning of the two arrays is the same, i.e. the two arrays are positioned in the same anatomical region close to each other. The breathing curves 430 of the marker array and the fiducial array deviate from each other in the amplitude of the phases of the breathing curves 430, that is in the deviation Δx from the reference position at Δx=0at specific points in time. In particular, in the present example, the breathing curves 430 of the marker array and the fiducial array deviate from each other in the deviation Δx from the respective reference position 440.

The above described second step 320 is repeated in a third step 330 for the patient tracker device 160 and the auto-registration device 150 being covered with a sterile drape, that is in a draped state. Thus, in preparation for this third step 330, a medical person covers the two devices 150, 160 with a sterile drape in step 325.

An abstract representation of first and second breathing movement patterns 510, 520 for the fiducial array and the marker array, respectively, in the draped state is shown in Figure 6. The respective breathing curves 530 in the draped state and the undraped state are the same as in the undraped state as regards the time aspect. That is, for example, the duration of a breathing cycle is the same or substantially the same in the draped sate of the fiducial array compared to the undraped state of the fiducial array, and in the draped state of the marker array compared to the undraped state of the marker array. However, due to tracking inaccuracies caused by possible wrinkles in the drape, the minimum and maximum of the breathing curve 530 in the draped state has changed compared to the breathing curve 430 in the undraped state. In particular, in the draped state, the breathing amplitudes are smaller than in the undraped state.

After the breathing movement patterns 410, 420, 510, 520 of the fiducial array and the marker array have been obtained, both for the draped state and the undraped state, an offset 540 (cf. Figure 7B) between the breathing movement patterns 410 and 510 in the undraped state and draped state of the auto-registration device 150 is calculated, and an offset 550 (cf. Figure 7A) between the breathing movement patterns 420 and 520 in the undraped state and draped state of the patient tracker device 160 is calculated. The determination of the offset 540, 550 may be performed by the data processing unit 130 of the surgical registration system 100.

For calculating the offset 540, 550, the respective breathing curves 430, 530 in the undraped und draped states have to be matched. This is achieved by matching the breathing phases 450, 460, 470, 480 of the breathing curves 430, 530 in the undraped und draped states. Since the breathing curves 430, 530 have their lowest point or inflection point in the exhaled state or phase 480 (no air in the lungs), this time interval may be used to match the breathing curves 430, 530.

An example of matched breathing curves 430, 530 is shown in Figures 7A and 7B, wherein Figure 7A shows the matched breathing curves 430, 530 for the patient's marker array of the patient tracker device 160 and Figure 7B shows the matched breathing curves 430, 530 for the fiducial array of the auto-registration device 150. The respective breathing curves 430, 530 are matched in the exhaled phase 480 having the lowest point in the respective breathing curve 430, 530. As is seen in Fig-ure 7A and 7B, the offset 540, 550 between the two breathing curves 430, 530 is determined for the respective positions of the tracked array in the breathing phase 460 where the lungs are full of air, i.e. in the inhaled state or phase 460 where the positional deviation Δx from the exhaled state 480 is maximum. Since in reality the breathing curve 430, 530 in the inhaled state 460 is not flat (different from the schematical illustration of Figures 7A and 7B), as can be also seen in Figure 4 of the drawings, the mean deviation of the positional deviation Δx in the inhaled state or phase 460 may be determined for each of the breathing curves 430, 530 in the draped and undraped states, and after that, the difference in the respective mean deviations may be determined for determining the offset 540, 550 in the exhaled state or phase 480 between the respective breathing curves 430, 530 in the draped state and undraped state.

An alternative method for determining an offset 550 between the respective breathing curves 430, 530 in the undraped and draped states comprises the steps of determining an end point of the respective gradient of the inhaling phase 450 (i.e. a point where the gradient transitions into the upper plateau shown in Figures 5 and 6 reflecting the state 460 where the lungs are full of air, i.e. the inhaled state 460), determining a start point of the respective gradient of the exhaling phase 470 (i.e. a point where the inhaled state or phase 460 transitions into the gradient of the exhaling phase 470) and then determining a respective center point between the determined end point and the determined start point. Then, the offset 550 can be calculated by subtracting values of the positional deviation Δx at these two center points, i.e. the center point determined for the draped curve and the center point determined for the undraped curve of the same tracker device.

As a further alternative method, it is possible to use machine learning, for example a neural network, for the pattern recognition of the respective breathing curves 430, 530 and determining the offset 550.

Generally, an offset 540, 550 can be determined for each breathing phase 450, 460, 470, 480 of the breathing movement patterns 410 and 510, and 420 and 520, and, in particular, for each point in time of a respective breathing phase 450, 460, 470, 480 after the breathing curves 430, 530 have been matched in time.

The determined offset 540, 550 may be used when comparing positional data obtained by detecting an array in a draped state with positional data obtained by detecting the array in the undraped state. In that case, the determined offset 540, 550 may be used for compensating a difference in the determined positional deviations Δx which is only caused by the different draping states (cf. Figures 7A and 7B). As a result, breathing curves 540, 550 obtained in draped and undraped states can be compared with each other.

The determined breathing patterns 410, 420, 510, 520 as exemplarily depicted in Figures 4 to 6 may be used to detect a specific breathing state at which the 3D medical image was captured. They may be further used to detect this specific breathing state at a later point in time for registering the captured 3D medical image data within the coordinate system of the surgical registration system 100 by using the auto-registration device 150 and, after that, to perform a tracker transfer from the fiducial array of the auto-registration device 150 to the marker array of the patient tracker device 160 at the same respiratory state at which the 3D medical image has been captured. Furthermore, they may be used to detect this specific breathing state when re-registering the captured 3D medical image data (or 3D medical image data captured at a later point in time, for example intra-operatively) within the coordinate system of the surgical registration system 100 by using the patient tracker device 160.

A tracker transfer from the fiducial array of the auto-registration device 150 to the marker array of the patient tracker device 160 may require determining relative positional data between the marker array and the radiopaque beads of the auto-registration device 150. The relative positional data may be determined by determining a relative position between the marker array of the patient tracker device 160 and the fiducial array of the auto-registration device 150 and using the pre-known relative position between the fiducial array and the radiopaque beads. In an embodiment, the relative position between the marker array of the patient tracker device 160 and the fiducial array of the auto-registration device 150 is determined by correlating positional data of the two tracker arrays detected at the same point in time or breathing phase of a breathing cycle, and in particular the point in time or breathing phase or breathing cycle where the 3D image has been captured.

Specifically, as is shown in Figure 3 of the drawings, in step 340, the patient's respiration may be manually stopped by a user such as the surgeon or the anesthetist, preferably when the detected breathing movement corresponds to the movement (i.e. position deviation Δx) of a specific, predetermined breathing phase 450, 460, 470, 480. Breathing is often stopped in the exhaled phase 480.

In step 350, as is seen in Figure 3, the data processing unit 130 is configured to recognize that the patient's breathing is stopped. In particular, in step 350, the data processing unit 350 may automatically, without any user input, detect that the patient's breathing is paused since no breathing movement is observed during a specific time frame. In particular, when recording the movement patterns of the tracker devices 150, 160, no change in positional deviation Δx is detected during the specific time frame when breathing has been stopped. As a consequence, i.e. when it is detected that breathing has been stopped, recording of the movement patterns of the auto-registration device 150 and the patient tracker device 160 is also stopped, automatically by the surgical navigation system 100, in step 355. In that manner, also the positional data of the patient tracker device 160 and the auto-registration device 150 during the paused breathing are captured and known, wherein, during the paused breathing, also a 3D scan is run.

In particular, the subsequent 3D scan for capturing a 3D image of the patient may be manually started by the responsible person such as the surgeon, as is shown in step 360. In that way, the 3D image is captured at a defined state of the breathing cycle, which can be, at a later point in time, re-identified by means of the known value for the positional deviation Δx at this defined state, both for the auto-registration device 150 and the patient tracker device 160.

In the second alternative, instead of manually stopping the patient's respiration by the user, it may be automatically stopped by the data processing unit 130 when the detected breathing movement corresponds to the movement (i.e. position deviation Δx) of the specific, predetermined breathing phase 450, 460, 470, 480. The subsequent steps may be the same as described above with respect to the first alternative.

Furthermore, instead of manually or automatically stopping the patient's respiration at a specific, predefined breathing phase 450, 460, 470, 480, it is also possible to manually or automatically stop the patient's respiration at any point in time within a breathing cycle. Since the data processing unit 130 is configured to recognize and record the respiratory state at which the patient's breathing is stopped, also in this variant, a subsequent registering process may be performed at the respiratory state at which the respiration has been stopped and the 3D image has been captured.

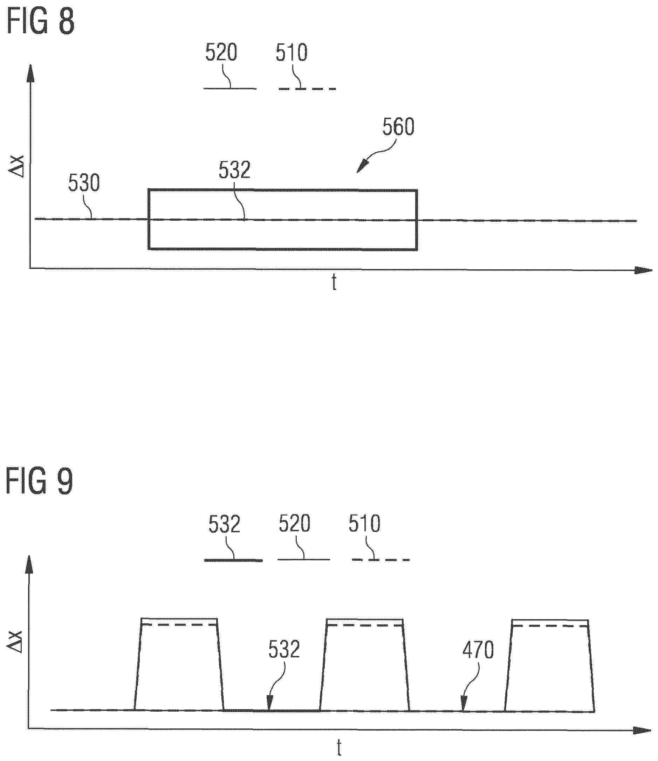

An exemplary abstraction of the breathing movement curves 530 of the patient tracker device 160 and the auto-registration device 150 is shown in Figure 8 of the drawings. As is illustrated, since the respiration is stopped during a time period 560 when the patient's body is scanned by means of the scanning unit 120, the breathing curves 530 are constant during the time period 560, i.e. the positional deviation Δx from, for example, the exhaled position 460 is constant for each of the patient tracker device 160 and the auto-registration device 150 during the time period 560. The positional deviation Δx for each of the patient tracker device 160 and the auto-registration device 150 during the time period 560 is designated with the reference numeral 532.

Figure 9 of the drawings shows how the respiratory state at which the patient's breathing has been stopped is determined by the data processing unit 130. In particular, as is seen in this figure, the determined and recorded positional data 532 in the draped state when the respiration has been stopped are compared with the pre-recorded breathing movement patterns 510, 520 of the patient tracker device 160 and the auto-registration device 150 in the draped state. In the present exemplary case, the data matching or comparison brings the result that the patient's respiration has been stopped in the exhaled state or phase 470 of a breathing cycle. In the alternative, also this step may be performed by machine learning, for example by using a neural network.

After the 3D scan has been finished, the patient's respiration is restarted in step 370 (cf. Figure 3), manually or automatically, and the patient is undraped in step 380 (cf. Figure 3).

Using the respiratory offset 540, 550 of each tracked device 150, 160 between the draped state and the undraped state, and knowing the respiratory state during the 3D scan, registering the 3D scan with the coordinate system of the surgical registration system 100 and handing over the registration from the fiducial array of the auto-registration device 150 to the marker array of the patient tracker device 160 at the respiratory state of the 3D scan can be performed.

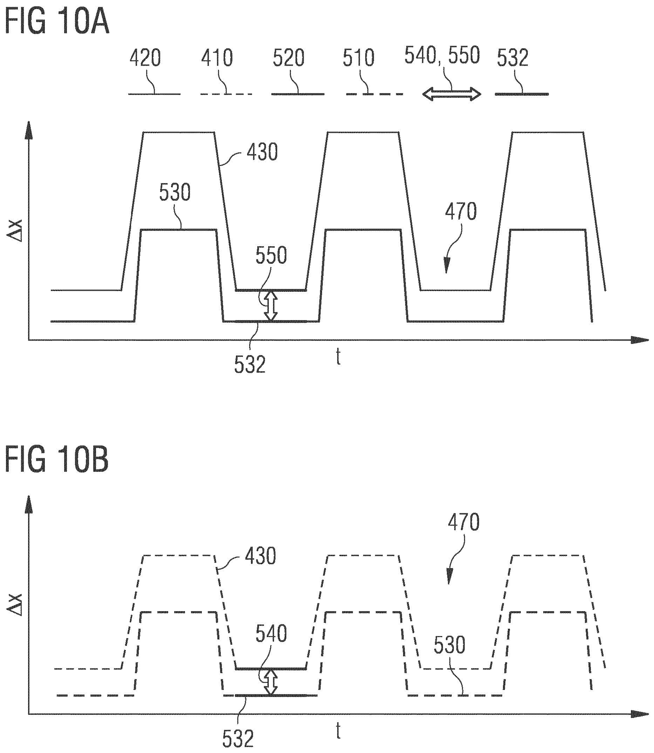

Figures 10A and 10B of the drawings schematically show how the breathing phase which corresponds to the breathing phase at which the 3D scan has run is determined from the current breathing movement pattern 410 in an undraped state.

As explained above with reference to Figure 9, based on the matching of the recorded positional data 532 when the patient's breathing has been stopped, which correspond to the positional data during the 3D scan, with the pre-recorded breathing movement patterns 510, 520 in a draped state, the breathing phase 470 at which the 3D scan has been performed is determined.

For detecting the corresponding breathing phase from the current breathing movement pattern 410, 420 in the undraped state, the positional deviation 532 during the 3D scan is "corrected" by the offset 540, 550 pre-determined for the respective breathing phase 470, as has been explained with reference to Figures 7A and 7B.

Figure 10A shows the offset 550 between the positional deviation Δx in the exhaled state/during the exhaled phase 470 for the marker array of the patient tracker device 160 in a draped state and an undraped state, and Figure 10B shows the offset 540 between the positional deviation Δx in the exhaled state/during the exhaled phase 470 for the fiducial array of the auto-registration device 150 in a draped state and an undraped state.

For detecting the exhaled state 470 when the patient is undraped before registering the captured medical image data within the coordinate system of the surgical registration system 100 in the detected exhaled state 470, the positional deviation data Δx (designated with reference numeral 532) during the 3D scan may be "corrected" by the offset 540 of Figure 10B determined for the fiducial array of the auto-registration device 150 thereby obtaining "corrected" positional deviation data Δx_{corr} for the fiducial array of the auto-registration device 150 during the 3D scan. This "corrected" positional deviation data Δx_{corr} for the fiducial array of the auto-registration device 150 may be used to determine the exhaled state in a currently recorded breathing movement pattern of the fiducial array, by a corresponding matching procedure with positional data of the currently determined breathing curve 430 describing the breathing movement of the auto-registration device 150.

Likewise, for detecting the exhaled state 470 when the patient is undraped before handing over the registration from the auto-registration device 150 to the patient tracker device 160 in the detected exhaled state, the positional deviation data Δx during the 3D scan may be "corrected" by the offset of Figure 10A determined for the patient tracker device 160 thereby obtaining "corrected" positional deviation data Δx_{corr} for the patient tracker device 160 during the 3D scan. This "corrected" positional deviation data Δx_{corr} for the patient tracker device 160 is used to determine the exhaled state in a currently recorded breathing movement pattern of the patient tracker device 160, by a corresponding matching procedure with positional data of the currently determined breathing curve 430 describing the breathing movement of the patient tracker device 160.

In the alternative, the above described breathing state detection may be performed by machine learning, for example using a neural network.

In particular, in the present example embodiment, for detecting the exhaled state 480 when the patient is undraped, before registering and/or re-registering the captured medical image data within the coordinate system of the surgical registration system 100 in the detected exhaled state 480 and before handing over the registration from the auto-registration device 150 to the patient tracker device 160 in the detected exhaled state 480, the matching procedure with respect to the breathing movement pattern 420 of the patient tracker device 160 and/or the matching procedure with respect to the breathing movement pattern 410 of the auto-registration device 150 may be performed.

Once the respiratory state of the 3D scan has been detected in the currently recorded breathing movement pattern 410, 420, the following two ways are possible for performing the registering process and/or re-registering process of the medical image data and/or the process of handing over registration data from the fiducial array to the patient tracker device.

In a first, manual way, the output device 140 of the surgical registration system 100 provides feedback to the user when the respiratory state during the 3D scan is aligned with or within a defined threshold of the current respiratory state. The user may then stop the respiration and manually start the registering process and/or the handing over process by using the current positional data of the optical markers 180 of the patient tracker device and, in a handing over process, the current positional data of the fiducial markers 220 of the auto-registration device 150 (in addition to the pre-known spatial relationship between the optical markers 180 and the radiopaque reference markers).

In a second, automatic way, the data processing device detects the aligned breathing state between the 3D scan and the current breathing state. The data processing device then automatically starts the registering process and/or the tracker transfer to ensure optical alignment between the breathing state of the 3D scan and the current breathing state.

Instead of using the current breathing movement pattern of the patient tracker device 160 and, optionally, of the auto-registration device 150 for registering the optical markers 180 or the fiducial markers 220 within the medical image data of the 3D scan, it is also possible to store or cache all navigation data and to use the stored or cached navigation data for calculating, by the data processing unit 130, the registering data required for the registering process. For example, in such an embodiment, the positional data of a pre-recorded breathing pattern 420, 410 of the patient tracker device 160 and, optionally of the auto-registration device 150, in the undraped state corresponding to the respiratory state during the 3D scan are used for calculating, by the data processing unit 130, the registering data required for the registering process (in addition to the pre-known spatial relationship between the optical markers 180 and the radiopaque reference markers), instead of the above-mentioned current positional data of the optical markers 180 of the patient tracker device and, in a handing over process, the current positional data of the fiducial markers 220 of the auto-registration device 150. Furthermore, also in this embodiment, the step of determining the breathing state at which the 3D scan has run as described with reference to Figure 9 is performed beforehand, as well as the subsequent step of determining the positional data of a pre-recorded breathing pattern 420, 410 of the patient tracker device 160 and, optionally of the auto-registration device 150, in the undraped state corresponding to the determined breathing state during the 3D scan by means of the pre-determined offset in the determined breathing state.

In Figures 1 to 10, an example has been described where the optical tracking device comprises the auto-registration device 150 and the patient tracker device 160. However, the present disclosure encompasses every configuration where an optical tracking device is attached to or held to or on a patient so that there might arise a positional mismatch between a medical image captured at a specific breathing phase and a subsequent registering process performed at a breathing phase different from the specific breathing phase. For example, the present disclosure also applies to the navigation system described in the "background art" of the present application, having a tracker permanently attached to the patient in combination with a tracker attached to a scanner, for example a tracker attached to a C-arm of an X-ray apparatus. For example, in this configuration, the "optical tracking device" of the present application comprises the tracker permanently attached to the patient, and the "medical imaging unit" of the present application comprises the X-ray apparatus.

Summarizing, the present disclosure provides a system and a method which allow to find and match the same point in time during a breathing cycle for the 3D scan and a subsequent registering process and/or process for transferring from a first tracker device to a second tracker device. Due to this it is possible to minimize the accuracy error of registration which is introduced during scanning and the actual registration thereafter. In addition, the present disclosure provides a solution how to compensate with the inaccuracies caused by comparing data recorded in a draped state with those recorded in an undraped state.

## Claims

1. A surgical registration system (100), comprising
- an optical tracking device (150, 160) associated with a patient;
- an optical detector unit (110) configured to detect the optical tracking device (150, 160) and to output a signal representative of a position of the optical tracking device (150, 160);
- a data processing unit (130) configured to determine a breathing movement pattern (410, 420, 510, 520) of the optical tracking device (150, 160) based on the output signal, the breathing movement pattern (410, 420, 510, 520) comprising positional data of the optical tracking device (150, 160) assigned to respective points in time of a time period, the breathing movement pattern (410, 420, 510, 520) defining a plurality of breathing phases (450, 460, 470, 480) of a breathing cycle; and
- a medical imaging unit (120) configured to capture a 3D image of the patient at a specific breathing phase (450, 460, 470, 480) of the breathing cycle,
wherein the data processing unit (130) is further configured to perform, at the specific breathing phase (450, 460, 470, 480) of the breathing cycle, a registration process for correlating a position of the 3D image with a corresponding patient's position.

2. The system (100) of claim 1, wherein the data processing unit (130) is configured to start or to cause indication of a start of the registration process when the positional data of the breathing movement pattern (410, 420, 510, 520) of the optical tracking device (150, 160) based on a current detection by the optical detector unit (110) corresponds to the positional data of the optical tracking device (150, 160) when capturing the 3D image.

3. The system (100) of claim 1 or 2, wherein the data processing unit (130) is configured to determine the specific breathing phase (450, 460, 470, 480) of the breathing cycle by comparing the breathing movement pattern (410, 420, 510, 520) of the optical tracking device (150, 160) based on a previous detection by the optical detector unit (110) with positional data of the optical tracking device (150, 160) when capturing the 3D image, wherein, optionally, the positional data at the specific breathing phase (450, 460, 470, 480) of the breathing movement pattern (410, 420, 510, 520) of the optical tracking device (150, 160) based on a previous detection by the optical detector unit (110) corresponds to the positional data of the optical tracking device (150, 160) when capturing the 3D image.

4. The system (100) of any of the preceding claims, wherein the breathing movement pattern (410, 420, 510, 520) comprises a draped breathing movement pattern (510, 520) determined in a draped state of the patient and an undraped breathing movement pattern (410, 420) determined in an undraped state of the patient, and wherein the data processing unit (130) is configured to determine an offset (540, 550) between the draped breathing movement pattern (510, 520) and the undraped breathing movement pattern (410, 420), the offset (540, 550) being related to a breathing phase (450, 460, 470, 480) of a breathing cycle.

5. The system (100) of claim 4, wherein the data processing unit (130) is configured to start or to cause indication of a start of the registration process when the positional data of the undraped breathing movement pattern (410, 420) of the optical tracking device (150, 160) based on a current detection by the optical detector unit (110) corresponds to corrected positional data of the optical tracking device (150, 160), wherein the corrected positional data corresponds to the positional data of the optical tracking device (150, 160) in the draped state of the patient when capturing the 3D image corrected by the offset (540, 550) between the draped breathing movement pattern (510, 520) and the undraped breathing movement pattern (410, 420) related to the specific breathing phase (450, 460, 470, 480) of the breathing cycle.

6. The system (100) of any of claims 2 to 5, wherein the positional data of the optical tracking device (150, 160) when capturing the 3D image corresponds to the positional data determined based on a previous detection by the optical detector unit (110) at a point in time where a patient's breathing has been stopped.

7. The system (100) of any of the preceding claims, wherein the optical tracking device (150, 160) comprises an auto-registration device (150) having a plurality of optical fiducial markers (220) forming a first array and a plurality of radiopaque reference markers forming a second array, wherein the plurality of radiopaque reference markers is detectable by the medical imaging unit (120) and the plurality of optical fiducial markers (220) is detectable by the optical detector unit (110), wherein, optionally, the plurality of radiopaque reference markers is integrated into one or more blades (260) that are configured to be pressed into the patient's body for placing the auto-registration device (150) on the patient.

8. The system (100) of any of the preceding claims, wherein the optical tracking device (150, 160) comprises a patient tracker device (160) having a plurality of optical markers (180) forming a third array and detectable by the optical detector unit (110).

9. The system (100) of claim 7 or 8, wherein the breathing movement pattern (410, 420, 510, 520) comprises at least one of a first breathing movement pattern (410, 510) of the auto-registration device (150) and a second breathing movement pattern (420, 520) of the patient tracker device (160).

10. The system (100) of claim 7 and optionally on claim 8 or 9, wherein the registration process correlates a position of the plurality of radiopaque reference markers forming the second array in the 3D image captured by the medical imaging unit (120) with a position of the plurality of optical fiducial markers (220) forming the first array determined based on the output signal of the optical detector unit (110).

11. The system (100) of claim 10 when dependent on claim 8 or 9, wherein the registration process correlates the position of the plurality of radiopaque reference markers forming the second array in the 3D image captured by the medical imaging unit (120) with a position of the plurality of optical markers (180) of the patient tracker device (160) forming the third array determined based on the output signal of the optical detector unit (110).

12. The system (100) of claim 11, wherein the data processing unit (130) is configured to determine a relative position between the plurality of optical markers (180) of the patient tracker device (160) and the plurality of optical fiducial markers (220) of the auto-registration device (150) at the specific breathing phase (450, 460, 470, 480) of the breathing cycle.

13. A method of performing a data registration process in a surgical registration system, the surgical registration system comprising an optical detector unit, a data processing unit, and a medical imaging unit, the method comprising the steps of
- detecting (320, 330), by the optical detector unit, an optical tracking device associated with a patient and outputting a signal representative of a position of the optical tracking device;
- determining (320, 330), by the data processing unit, a breathing movement pattern of the optical tracking device based on the output signal, the breathing movement pattern comprising positional data of the optical tracking device assigned to respective points in time of a time period, the breathing movement pattern defining a plurality of breathing phases of a breathing cycle;
- capturing (360), by the medical imaging unit, a 3D image of the patient at a specific breathing phase of the breathing cycle while detecting the optical tracking device; and
- performing, by the data processing unit, at the specific breathing phase of the breathing cycle, a registration process for correlating a position of the 3D image with a corresponding patient's position.

14. The method of claim 13, comprising the step of starting or causing indication of a start of the registration process, by the data processing unit, when the positional data of the first breathing movement pattern of the optical tracking device based on a current detection by the optical detector unit corresponds to the positional data of the optical tracking device when capturing the 3D image.

15. The method of claim 13 or 14, comprising the step of determining, by the data processing unit, the specific breathing phase of the breathing cycle by comparing a breathing movement pattern of the optical tracking device based on a previous detection by the optical detector unit with positional data of the optical tracking device when capturing the 3D image, wherein, optionally, the positional data at the specific breathing phase of the breathing movement pattern of the optical tracking device based on the previous detection by the optical detector unit corresponds to the positional data of the optical tracking device when capturing the 3D image.

16. The method of any of claims 13 to 15, wherein the breathing movement pattern comprises a draped breathing movement pattern determined in a draped state of the patient and an undraped breathing movement pattern determined in an undraped state of the patient, and wherein the data processing unit determines an offset between the draped breathing movement pattern and the un-draped breathing movement pattern, the offset being related to a breathing phase of a breathing cycle.

17. The method of claim 16, comprising the step of starting or causing indication of a start of the registration process, by the data processing unit, when the positional data of the undraped breathing movement pattern of the optical tracking device based on a current detection by the optical detector unit corresponds to corrected positional data of the optical tracking device, wherein the corrected positional data corresponds to the positional data of the optical tracking device when capturing the 3D image corrected by the offset between the draped breathing movement pattern and the undraped breathing movement pattern related to the specific breathing phase of the breathing cycle.

18. The method of any claims 13 to 17, wherein, during the registration process, a position of a plurality of radiopaque reference markers forming a second array in the 3D image captured by the medical imaging unit is correlated with a position of a plurality of optical fiducial markers forming a first array determined based on the output signal of the optical detector unit, wherein the plurality of radiopaque reference markers is detectable by the scanning device and the plurality of optical fiducial markers is detectable by the optical detector unit, and wherein, optionally, the optical tracking device comprises an auto-registration device having the plurality of optical fiducial markers forming the first array and the plurality of radiopaque reference markers forming the second array.

19. The method of claim 18, wherein, during the registration process, the position of the plurality of radiopaque reference markers forming the second array in the 3D image captured by the medical imaging unit is correlated with a position of a plurality of optical markers forming a third array determined based on the output signal of the optical detector unit, wherein the plurality of optical markers forming the third array is detectable by the optical detector unit, and wherein, optionally, the optical tracking device comprises a patient tracker device having the plurality of optical markers forming the third array.

20. The method of claim 19, comprising the step of determining a relative position between the plurality of optical markers of the patient tracker device and the plurality of optical fiducial markers of the auto-registration device at the specific breathing phase of the breathing cycle.

## Amended claims

### Amended claims in accordance with Rule 137(2) EPC.

1. A surgical registration system (100), comprising
- an optical tracking device (150, 160) associated with a patient;
- an optical detector unit (110) configured to detect the optical tracking device (150, 160) and to output a signal representative of a position of the optical tracking device (150, 160);
- a data processing unit (130) configured to determine a breathing movement pattern (410, 420, 510, 520) of the optical tracking device (150, 160) based on the output signal, the breathing movement pattern (410, 420, 510, 520) comprising positional data of the optical tracking device (150, 160) assigned to respective points in time of a time period, the breathing movement pattern (410, 420, 510, 520) defining a plurality of breathing phases (450, 460, 470, 480) of a breathing cycle; and
- a medical imaging unit (120) configured to capture a 3D image of the patient at a specific breathing phase (450, 460, 470, 480) of the breathing cycle,
wherein the data processing unit (130) is further configured to determine the specific breathing phase (450, 460, 470, 480) of the breathing cycle by comparing the breathing movement pattern (410, 420, 510, 520) of the optical tracking device (150, 160) based on a previous detection by the optical detector unit (110) with positional data of the optical tracking device (150, 160) when capturing the 3D image and to perform, at the determined specific breathing phase (450, 460, 470, 480) of the breathing cycle, a registration process for correlating a position of the 3D image with a corresponding patient's position.

2. The system (100) of claim 1, wherein the data processing unit (130) is configured to start or to cause indication of a start of the registration process when the positional data of the breathing movement pattern (410, 420, 510, 520) of the optical tracking device (150, 160) based on a current detection by the optical detector unit (110) corresponds to the positional data of the optical tracking device (150, 160) when capturing the 3D image.

3. The system (100) of claim 1 or 2, wherein the positional data at the specific breathing phase (450, 460, 470, 480) of the breathing movement pattern (410, 420, 510, 520) of the optical tracking device (150, 160) based on a previous detection by the optical detector unit (110) corresponds to the positional data of the optical tracking device (150, 160) when capturing the 3D image.

4. The system (100) of any of the preceding claims, wherein the breathing movement pattern (410, 420, 510, 520) comprises a draped breathing movement pattern (510, 520) determined in a draped state of the patient and an undraped breathing movement pattern (410, 420) determined in an undraped state of the patient, and wherein the data processing unit (130) is configured to determine an offset (540, 550) between the draped breathing movement pattern (510, 520) and the undraped breathing movement pattern (410, 420), the offset (540, 550) being related to a breathing phase (450, 460, 470, 480) of a breathing cycle.

5. The system (100) of claim 4, wherein the data processing unit (130) is configured to start or to cause indication of a start of the registration process when the positional data of the undraped breathing movement pattern (410, 420) of the optical tracking device (150, 160) based on a current detection by the optical detector unit (110) corresponds to corrected positional data of the optical tracking device (150, 160), wherein the corrected positional data corresponds to the positional data of the optical tracking device (150, 160) in the draped state of the patient when capturing the 3D image corrected by the offset (540, 550) between the draped breathing movement pattern (510, 520) and the undraped breathing movement pattern (410, 420) related to the specific breathing phase (450, 460, 470, 480) of the breathing cycle.

6. The system (100) of any of claims 2 to 5, wherein the positional data of the optical tracking device (150, 160) when capturing the 3D image corresponds to the positional data determined based on a previous detection by the optical detector unit (110) at a point in time where a patient's breathing has been stopped.

7. The system (100) of any of the preceding claims, wherein the optical tracking device (150, 160) comprises an auto-registration device (150) having a plurality of optical fiducial markers (220) forming a first array and a plurality of radiopaque reference markers forming a second array, wherein the plurality of radiopaque reference markers is detectable by the medical imaging unit (120) and the plurality of optical fiducial markers (220) is detectable by the optical detector unit (110), wherein, optionally, the plurality of radiopaque reference markers is integrated into one or more blades (260) that are configured to be pressed into the patient's body for placing the auto-registration device (150) on the patient.

8. The system (100) of any of the preceding claims, wherein the optical tracking device (150, 160) comprises a patient tracker device (160) having a plurality of optical markers (180) forming a third array and detectable by the optical detector unit (110).

9. The system (100) of claim 8 when dependent on claim 7, wherein the breathing movement pattern (410, 420, 510, 520) comprises at least one of a first breathing movement pattern (410, 510) of the auto-registration device (150) and a second breathing movement pattern (420, 520) of the patient tracker device (160).

10. The system (100) of claim 7 and optionally on claim 8 or 9, wherein the registration process correlates a position of the plurality of radiopaque reference markers forming the second array in the 3D image captured by the medical imaging unit (120) with a position of the plurality of optical fiducial markers (220) forming the first array determined based on the output signal of the optical detector unit (110).

11. The system (100) of claim 10 when dependent on claim 8 or 9, wherein the registration process correlates the position of the plurality of radiopaque reference markers forming the second array in the 3D image captured by the medical imaging unit (120) with a position of the plurality of optical markers (180) of the patient tracker device (160) forming the third array determined based on the output signal of the optical detector unit (110).

12. The system (100) of claim 11, wherein the data processing unit (130) is configured to determine a relative position between the plurality of optical markers (180) of the patient tracker device (160) and the plurality of optical fiducial markers (220) of the auto-registration device (150) at the specific breathing phase (450, 460, 470, 480) of the breathing cycle.

13. A method of performing a data registration process in a surgical registration system, the surgical registration system comprising an optical detector unit, a data processing unit, and a medical imaging unit, the method comprising the steps of
- detecting (320, 330), by the optical detector unit, an optical tracking device associated with a patient and outputting a signal representative of a position of the optical tracking device;
- determining (320, 330), by the data processing unit, a breathing movement pattern of the optical tracking device based on the output signal, the breathing movement pattern comprising positional data of the optical tracking device assigned to respective points in time of a time period, the breathing movement pattern defining a plurality of breathing phases of a breathing cycle;
- capturing (360), by the medical imaging unit, a 3D image of the patient at a specific breathing phase of the breathing cycle; and
- determining, by the data processing unit, the specific breathing phase of the breathing cycle by comparing the breathing movement pattern of the optical tracking device based on a previous detection by the optical detector unit with positional data of the optical tracking device when capturing the 3D image, and performing, by the data processing unit, at the determined specific breathing phase of the breathing cycle, a registration process for correlating a position of the 3D image with a corresponding patient's position.

14. The method of claim 13, comprising the step of starting or causing indication of a start of the registration process, by the data processing unit, when the positional data of the first breathing movement pattern of the optical tracking device based on a current detection by the optical detector unit corresponds to the positional data of the optical tracking device when capturing the 3D image.

15. The method of claim 13 or 14, wherein the positional data at the specific breathing phase of the breathing movement pattern of the optical tracking device based on the previous detection by the optical detector unit corresponds to the positional data of the optical tracking device when capturing the 3D image.

16. The method of any of claims 13 to 15, wherein the breathing movement pattern comprises a draped breathing movement pattern determined in a draped state of the patient and an undraped breathing movement pattern determined in an un-draped state of the patient, and wherein the data processing unit determines an offset between the draped breathing movement pattern and the un-draped breathing movement pattern, the offset being related to a breathing phase of a breathing cycle.

17. The method of claim 16, comprising the step of starting or causing indication of a start of the registration process, by the data processing unit, when the positional data of the undraped breathing movement pattern of the optical tracking device based on a current detection by the optical detector unit corresponds to corrected positional data of the optical tracking device, wherein the corrected positional data corresponds to the positional data of the optical tracking device when capturing the 3D image corrected by the offset between the draped breathing movement pattern and the undraped breathing movement pattern related to the specific breathing phase of the breathing cycle.

18. The method of any claims 13 to 17, wherein, during the registration process, a position of a plurality of radiopaque reference markers forming a second array in the 3D image captured by the medical imaging unit is correlated with a position of a plurality of optical fiducial markers forming a first array determined based on the output signal of the optical detector unit, wherein the plurality of radiopaque reference markers is detectable by the scanning device and the plurality of optical fiducial markers is detectable by the optical detector unit, and wherein, optionally, the optical tracking device comprises an auto-registration device having the plurality of optical fiducial markers forming the first array and the plurality of radiopaque reference markers forming the second array.

19. The method of claim 18, wherein, during the registration process, the position of the plurality of radiopaque reference markers forming the second array in the 3D image captured by the medical imaging unit is correlated with a position of a plurality of optical markers forming a third array determined based on the output signal of the optical detector unit, wherein the plurality of optical markers forming the third array is detectable by the optical detector unit, and wherein, optionally, the optical tracking device comprises a patient tracker device having the plurality of optical markers forming the third array.

20. The method of claim 19, comprising the step of determining a relative position between the plurality of optical markers of the patient tracker device and the plurality of optical fiducial markers of the auto-registration device at the specific breathing phase of the breathing cycle.
